(19) Europäisches Patentamt
European Patent Office
Office européen des brevets

(11) Publication number: **0 090 262 B1**

(12) **EUROPEAN PATENT SPECIFICATION**

(45) Date of publication of patent specification: **05.08.92**

(51) Int. Cl.[5]: **C07C 49/813**, C07C 49/84, C07C 205/12, A01N 35/06

(21) Application number: **83102599.4**

(22) Date of filing: **16.03.83**

(54) **Certain 2-(2-substituted benzoyl)-1,3-cyclohexanediones.**

(30) Priority: **09.02.83 US 464251**
**25.03.82 US 361658**

(43) Date of publication of application:
**05.10.83 Bulletin 83/40**

(45) Publication of the grant of the patent:
**05.08.92 Bulletin 92/32**

(84) Designated Contracting States:
**AT BE CH DE FR GB IT LI NL SE**

(56) References cited:
**EP-A- 0 007 243**
**EP-A- 0 017 195**

**CHEMICAL ABSTRACTS, vol. 85, 1976, page 423, no. 5280f, Columbus, Ohio, US**

**SYNTHESIS, December 1978, pages 925-926, Georg Thieme Publishers A.A. AKHREM et al.: "A new simple synthesis of 2-acylcyclohexane-1,3-diones"**

**CHEMICAL ABSTRACTS, vol. 84, 1976, page 174, no. 175144m, Columbus, Ohio, US**

(73) Proprietor: **STAUFFER CHEMICAL COMPANY**

**Westport Connecticut 06880(US)**

(72) Inventor: **Michaely, William James**
**3161 Birmingham Drive**
**Richmond, Ca. 94806(US)**
Inventor: **Kraatz, Gary Wayne**
**1423 Bing Drive**
**San Jose, Ca. 95129(US)**

(74) Representative: **Kraus, Walter, Dr. et al**
**Patentanwälte Kraus, Weisert & Partner**
**Thomas-Wimmer-Ring 15**
**W-8000 München 22(DE)**

**EP 0 090 262 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Background of the Invention

Compounds having the structural formula

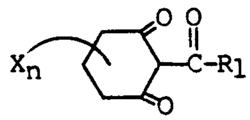

wherein X can be an alkyl, n can be 0, 1, or 2, and $R_1$ can be phenyl or substituted phenyl are described in Japanese Patent Application 84632-1974 as being intermediates for the preparation of herbicidal compounds of the formula

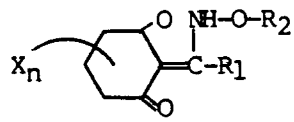

wherein $R_1$, X, and n are as defined above and $R_2$ is alkyl, alkenyl, or alkynyl. Specifically taught herbicidal compounds of this latter group are those where n is 2, X is 5,5-dimethyl, $R_2$ is allyl and $R_1$ is phenyl, 4-chlorophenyl or 4-methoxyphenyl.

The precursor intermediates for these three specifically taught compounds have no or almost no herbicidal activity.

In contrast, the compounds of this invention have exceptional herbicidal activity. Applicant's compounds must have a halogen substitution in the 2-position of the phenyl moiety of their compounds to obtain the exceptional herbicidal activity. The exact reason why such a substitution imparts exceptional herbicidal activity to the compound is not fully understood.

Description of the Invention

This invention relates to certain novel 2-(2-substituted benzoyl)-cyclohexane-1,3-diones as herbicides. The compounds of this invention have the following structural formula

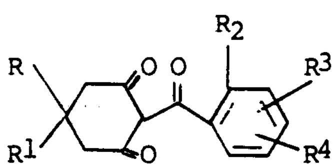

wherein R and $R^1$ are hydrogen or $C_1$-$C_4$ alkyl, preferably methyl, most preferably R and $R^1$ are hydrogen, $R^2$ is chlorine, bromine, or iodine, $R^3$ is hydrogen or halogen, preferably iodine or chlorine, most preferably $R^3$ is hydrogen, and $R^4$ is hydrogen, chlorine, bromine, iodine, $C_1$-$C_4$ alkyl, preferably methyl, $C_1$-$C_4$ alkoxy, preferably methoxy, nitro, trifluoromethyl, most preferably $R^4$ is hydrogen and 4-chlorine.

The compounds of this invention can have the following three structural formulae because of tautomerism:

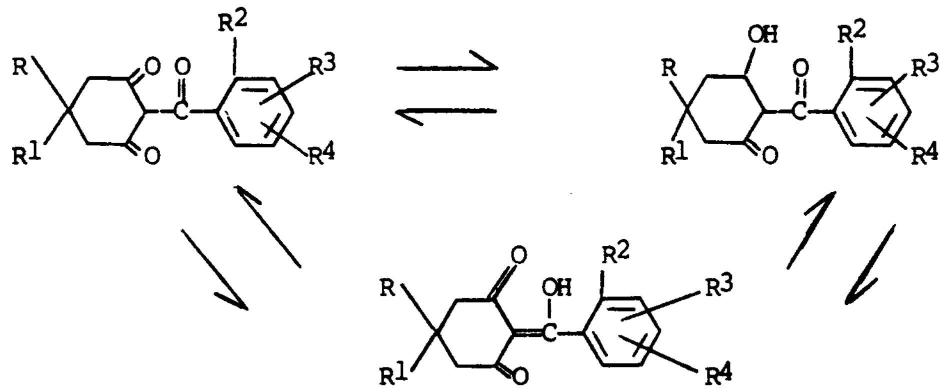

wherein R, $R^1$, $R^2$, $R^3$, and $R^4$ are as defined above.

In the above description of the compounds of this invention alkyl and alkoxy include both straight and branched configurations; for example, methyl ethyl, n-propyl, isopropyl, n-butyl, sec-butyl, isobutyl, and tert-butyl.

The compounds of this invention are active herbicides of a general type. That is, they are herbicidally effective against a wide range of plant species. The method of controlling undesirable vegetation of the present invention comprises applying an herbicidally effective amount of the above-described compounds to the area where control is desired.

The compounds of the present invention can be prepared by the following general method.

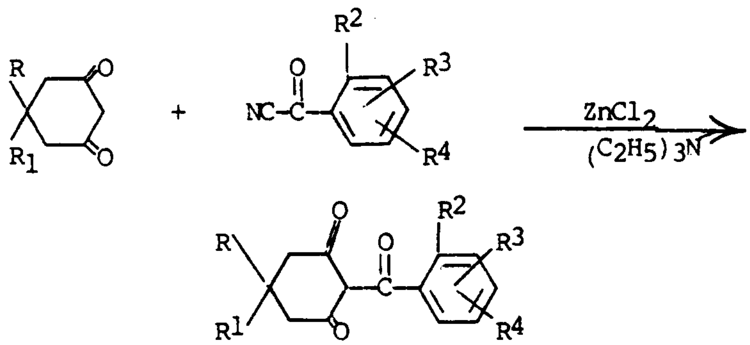


Generally, mole amounts of the dione and substituted benzoyl cyanide are used, along with a slight mole excess of zinc chloride. The two reactants and the zinc chloride are combined in a solvent such as methylene chloride. A slight mole excess of triethylamine is slowly added to the reaction mixture with cooling. The mixture is stirred at room temperature for 5 hours.

The reaction product is worked up by conventional techniques.

The above-described substituted benzoyl cyanide can be prepared according to the teaching of T.S. Oakwood and C.A. Weisgerber, Organic Synthesis Collected, Vol. III, pp. 122 (1955).

The following example teaches the synthesis of a representative compound of this invention.

EXAMPLE I

2-(2,4-Dichlorobenzoyl)-cyclohexane-1,3-dione

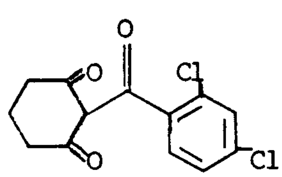

1,3-Cyclohexanedione [11.2 grams (g), 0.1 mole], 20.0 g (0.1 mole) 2,4-dichlorobenzoyl cyanide and 13.6 g (0.11 mole) anhydrous, powdered zinc chloride were combined in 100 milliliters (ml) methylene chloride. Triethylamine (10.1 g, 0.12 mole) was slowly added with cooling. The reaction mixture was stirred at room temperature for 5 hours and then poured into 2N hydrochloric acid. The aqueous phase was discarded and the organic phase was washed with 150 ml 5% $Na_2CO_3$ four times. The aqueous washings were combined and acidified with HCl, extracted with methylene chloride, dried and concentrated to yield 25.3 g of crude product. The crude product was dissolved in ether and stirred with 250 ml of 5% copper (II) acetate. The resulting copper salt was filtered, washed with ether and stirred with 6N hydrochloric acid to destroy the salt. The extract was washed with ether to yield 22.15 grams of the desired product m.p. 138-140 ° C. (77.7% yield). The structure was confirmed by instrumental analysis.

The following is a table of certain selected compounds that are preparable according to the procedure described hereto. Compound numbers are assigned to each compound and are used throughout the remainder of the application.

TABLE I

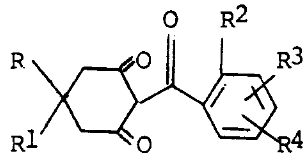

| Compound Number | R | $R^1$ | $R^2$ | $R^3$ | $R_4$ | $N_D^{30}$ or m.p. °C |
|---|---|---|---|---|---|---|
| 1 | $CH_3$ | $CH_3$ | 2-Cl | H | H | 1.5613 |
| 2 | $CH_3$ | $CH_3$ | 2-Cl | H | 4-Cl | 1.5655 |
| 3 | $CH_3$ | $CH_3$ | 2-Cl | H | 6-Cl | 103-108 |
| 4* | H | H | 2-Cl | H | 4-Cl | 138-140 |
| 5 | $CH_3$ | $CH_3$ | 2-Br | H | H | |
| 6 | $CH_3$ | $CH_3$ | 2-Cl | H | 5-Cl | 74-77 |
| 7 | H | H | 2-Cl | H | 5-Cl | 104-107 |
| 8 | H | H | 2-Br | H | H | 93-96 |
| 9 | H | H | 2-Cl | H | H | 79-87 |
| 10 | H | H | 2-I | H | H | 66-70 |
| 11 | H | H | 2-Cl | H | 4-$NO_2$ | 118-122 |
| 12 | H | H | 2-Cl | H | 6-Cl | 143-148 |
| 13 | H | H | 2-Cl | H | 5-Br | 109-115 |
| 14 | H | H | 2-I | 3-I | 5-I | 164-167 |
| 15 | H | H | 2-Cl | H | 5-$CH_3$ | 60-65 |
| 16 | H | H | 2-Cl | H | 4-$CH_3O$ | 79-86 |
| 17 | H | H | 2-Cl | H | 4-$CH_3$ | 60-63 |
| 18 | H | H | 2-Cl | 3-Cl | 6-Cl | |
| 19 | H | H | 2-Cl | H | 5-$CH_3O$ | 77-80 |
| 20 | H | H | 2-Cl | H | 3-Cl | 80-90 |
| 21 | H | H | 2-Cl | H | 5-$CF_3$ | 74-75 |
| 22 | H | H | 2-Cl | H | 5-$NO_2$ | 140-143 |
| 23 | H | H | 2-Cl | 3-Cl | 4-Cl | 152-154 |
| 24 | H | H | 2-Cl | 3-Cl | 4-$CH_3O$ | 169-170 |
| 25 | H | H | 2-Cl | H | 4-Br | 104-107 |

* Prepared in the example.

Herbicidal Screening Tests

As previously mentioned, the herein described compounds produced in the above-described manner are phytotoxic compounds which are useful and valuable in controlling various plant species. Selected compounds of this invention were tested as herbicides in the following manner.

Pre-emergence herbicide test. On the day preceding treatment, seeds of eight different weed species are planted in loamy sand soil in individual rows using one species per row across the width of a flat. The seeds used are green foxtail (FT) (*Setaria viridis*), watergrass (WG) (*Echinochloa crusgalli*), wild oat (WO)

(Avena fatua), annual morningglory (AMG) (Ipomoea lacunosa), velvetleaf (VL) (Abutilon theophrasti), Indian mustard (MD (Brassica juncea), redroot pigweed (PW) (Amaranthus retroflexus), and yellow nutsedge (YNG) (Cyperus esculentus). Ample seeds are planted to give about 20 to 40 seedlings per row, after emergence, depending upon the size of the plants.

Using an analytical balance, 600 milligrams (mg) of the compound to be tested are weighed out on a piece of glassine weighing paper. The paper and compound are placed in a 60 milliliter (ml) wide-mouth clear bottle and dissolved in 45 ml of acetone or substituted solvent. Eighteen ml of this solution are transferred to a 60 ml wide-mouth clear bottle and diluted with 22 ml of a water and acetone mixture (19:1) containing enough polyoxyethylene sorbitan monolaurate emulsifier to give a final solution of 0.5% (v/v). The solution is then sprayed on a seeded flat on a linear spray table calibrated to deliver 80 gallons per acre (748 L/ha). The application rate is 4 lb/acre (4.48 Kg/ha).

After treatment, the flats are placed in the greenhouse at a temperature of 70 to 80°F and watered by sprinkling. Two weeks after treatment, the degree of injury or control is determined by comparison with untreated check plants of the same age. The injury rating from 0 to 100% is recorded for each species as percent control with 0% representing no injury and 100% representing complete control.

The results of the tests are shown in the following Table II.

TABLE II

| Pre-Emergence Herbicidal Activity Application Rate -- 4.48 kg/ha | | | | | | | | |
|---|---|---|---|---|---|---|---|---|
| Cmpd. No. | FT | WG | WO | AMG | VL | MD | PW | YNG |
| 1 | 100 | 90 | 20 | 40 | 65 | 50 | 35 | 80 |
| 2 | 80 | 90 | 0 | 30 | 80 | 90 | 20 | 90 |
| 3 | 0 | 0 | 0 | 40 | 40 | 40 | 0 | 0 |
| 4 | 100 | 100 | 50 | 35 | 100 | 100 | 90 | 95 |
| 5 | 100 | 95 | 65 | 35 | 90 | 85 | 50 | 95 |
| 6 | 50 | 25 | 0 | 25 | 100 | 100 | 35 | 80 |
| 7 | 80 | 90 | 10 | 60 | 100 | 100 | 100 | 80 |
| 8 | 95 | 95 | 45 | 40 | 100 | 80 | 95 | 95 |
| 9 | 95 | 100 | 55 | 30 | 100 | 90 | 100 | 95 |
| 10 | 100 | 100 | 60 | 20 | 85 | 100 | 100 | 95 |
| 11 | 85 | 100 | 30 | 40 | 100 | 100 | 100 | 95 |
| 12 | 85 | 85 | 90 | 75 | 80 | 95 | 90 | 90 |
| 13 | 85 | 75 | 10 | 10 | 85 | 65 | 95 | 90 |
| 14 | 40 | 10 | 80 | 0 | 65 | 40 | 65 | 100 |
| 15 | 40 | 60 | 20 | 30 | 100 | 30 | 75 | 95 |
| 16 | 80 | 80 | 20 | 55 | 75 | 90 | 40 | 95 |
| 17 | 20 | - | 10 | 25 | 100 | 95 | 85 | 90 |
| 18 | 65 | - | 30 | 70 | 100 | 100 | 85 | 90 |
| 19 | 45 | 60 | 0 | 30 | 40 | 0 | 20 | 60 |
| 20 | 30 | 85 | 20 | 40 | 95 | 85 | 40 | 45 |
| 21 | 80 | 100 | 0 | 80 | 100 | 100 | 95 | 95 |
| 22 | 20 | 10 | 0 | 20 | 75 | 30 | 45 | 10 |
| 23 | 45 | 95 | 10 | 0 | 100 | 85 | 100 | 95 |
| 24 | - | 100 | 0 | 25 | 70 | 90 | - | 100 |
| 25 | - | 100 | 25 | 80 | 100 | 100 | - | 100 |
| - = Specie did not germinate for some unknown reason. | | | | | | | | |

Post-Emergence Herbicide Test: This test is conducted in an identical manner to the testing procedure for the pre-emergence herbicide test, except the seeds of the eight different weed species are planted 10-12 days before treatment. Also, watering of the treated flats is confined to the soil surface and not to the foliage of the sprouted plants.

The results of the post-emergence herbicide test are reported in Table III.

TABLE III

| Post-Emergence Herbicidal Activity Application Rate -- 4.48 kg/ha | | | | | | | | |
|---|---|---|---|---|---|---|---|---|
| Cmpd. No. | FT | WG | WO | AMG | VL | MD | PW | YNG |
| 1 | 60 | 70 | 20 | 40 | 60 | 60 | 35 | 60 |
| 2 | 30 | 70 | 0 | 50 | 90 | 85 | 30 | 80 |
| 3 | 0 | 30 | 0 | 70 | 100 | 90 | 55 | 70 |
| 4 | 95 | 98 | 20 | 98 | 100 | 100 | 30 | 95 |
| 5 | 80 | 80 | 75 | 50 | 60 | 80 | 0 | 95 |
| 6 | 40 | 40 | 10 | 60 | 100 | 100 | 75 | 65 |
| 7 | 60 | 75 | 40 | 60 | 100 | 75 | 100 | 75 |
| 8 | 85 | 80 | 75 | 70 | 95 | 80 | 90 | 90 |
| 9 | 85 | 80 | 75 | 70 | 95 | 80 | 90 | 90 |
| 10 | 95 | 85 | 90 | 60 | 95 | 95 | 80 | 95 |
| 11 | 50 | 80 | 35 | 55 | 100 | 100 | 95 | 80 |
| 12 | 45 | 75 | 50 | 55 | 75 | 60 | 50 | 80 |
| 13 | 30 | 60 | 20 | 60 | 80 | 50 | 60 | 70 |
| 14 | 20 | 10 | 20 | 50 | 45 | 40 | 40 | 0 |
| 15 | 65 | 95 | 0 | 65 | 95 | 30 | 100 | 80 |
| 16 | 65 | 80 | 20 | 85 | 85 | 30 | 30 | 80 |
| 17 | 75 | 80 | 30 | 70 | 100 | 100 | 85 | 90 |
| 18 | 100 | 95 | 10 | 100 | 100 | 100 | 100 | 90 |
| 19 | 60 | 80 | 40 | 70 | 100 | 75 | 80 | 90 |
| 20 | 65 | 80 | 10 | 85 | 95 | 95 | 100 | 70 |
| 21 | 30 | 55 | 0 | 80 | 100 | 80 | 65 | 80 |
| 72 | 0 | 30 | 0 | 20 | 45 | 0 | 30 | 20 |
| 23 | 85 | 90 | 40 | 85 | 100 | 95 | 100 | 90 |
| 24 | 0 | 80 | 0 | 70 | 90 | 74 | - | 100 |
| 25 | 100 | 100 | 75 | 90 | 100 | 100 | - | 100 |
| -- = Specie did not germinate for some reason. | | | | | | | | |

The compounds of the present invention are useful as herbicides, especially as pre-emergence herbicides, and can be applied in a variety of ways at various concentrations. In practice, the compounds herein defined are formulated into herbicidal compositions, by admixture, in herbicidally effective amounts, with the adjuvants and carriers normally employed for facilitating the dispersion of active ingredients for agricultural applications, recognizing the fact that the formulation and mode of application of a toxicant may affect the activity of the materials in a given application. Thus, these active herbicidal compounds may be formulated as granules of relatively large particle size, as wettable powders, as emulsifiable concentrates, as powdery dusts, as solutions or as any of several other known types of formulations, depending upon the desired mode of application. Preferred formulations for pre-emergence herbicidal applications are wettable powders, emulsifiable concentrates and granules. These formulations may contain as little as about 0.5% to as much as about 95% or more by weight of active ingredient. A herbicidally effective amount depends upon the nature of the seeds or plants to be controlled and the rate of application varies from about 0.05 to approximately 25 pounds per acre, preferably from about 0.1 to about 10 pounds per acre.

Wettable powders are in the form of finely divided particles which disperse readily in water or other dispersants. The wettable powder is ultimately applied to the soil either as a dry dust or as a dispersion in water or other liquid. Typical carriers for wettable powders include fuller's earth, kaolin clays, silicas and other readily wet organic or inorganic diluents. Wettable powders normally are prepared to contain about 5% to about 95% of the active ingredient and usually also contain a small amount of wetting, dispersing, or emulsifying agent to facilitate wetting and dispersion.

Emulsifiable concentrates are homogeneous liquid compositions which are dispersible in water or other dispersant, and may consist entirely of the active compound with a liquid or solid emulsifying agent, or may also contain a liquid carrier, such as xylene, heavy aromatic naphthal, isophorone and other non-volatile organic solvents. For herbicidal application, these concentrates are dispersed in water or other liquid carrier and normally applied as a spray to the area to be treated. The percentage by weight of the essential active ingredient may vary according to the manner in which the composition is to be applied, but in general

comprises about 0.5% to 95% of active ingredient by weight of the herbicidal composition.

Granular formulations wherein the toxicant is carried on relatively coarse particles, are usually applied without dilution to the area in which suppression of vegetation is desired. Typical carriers for granular formulations include sand, fuller's earth, bentonite clays, vermiculite, perlite and other organic or inorganic materials which absorb or which may be coated with the toxicant. Granular formulations normally are prepared to contain about 5% to about 25% of active ingredients which may include surface-active agents such heavy aromatic naphthas, kerosene or other petroleum fractions, or vegetable oils; and/or stickers such as destrins, glue or synthetic resins.

Typical wetting, dispersing or emulsifying agents used in agricultural formulations include, for example, the alkyl and alkylaryl sulfonates and sulfates and their sodium salts; polyhydric alcohols; and other types of surface-active agents, many of which are available in commerce. The surface-active agent, when used, normally comprises from 0.1% to 15% by weight of the herbicidal composition.

Dusts, which are free-flowing admixtures of the active ingredient with finely divided solids such as talc, clays, flours and other organic and inorganic solids which act as dispersants and carriers for the toxicant, are useful formulations for soil-incorporating application.

Pastes, which are homogeneous suspensions of a finely divided solid toxicant in a liquid carrier such as water or oil, are employed for specific purposes. These formulations normally contain about 5% to about 95% of active ingredient by weight, and may also contain small amounts of a wetting, dispersing or emulsifying agent to facilitate dispersion. For application, the pastes are normally diluted and applied as a spray to the area to be affected.

Other useful formulations for herbicidal applications include simple solutions of the active ingredient in a dispersant in which it is completely soluble at the desired concentration, such as acetone, alkylated naphthalenes, xylene and other organic solvents. Pressurized sprays, typically aerosols, wherein the active ingredient is dispersed in finely-divided form as a result of vaporization of a low boiling dispersant solvent carrier, such as the Freons, may also be used.

The phytotoxic compositions of this invention are applied to the plants in the conventional manner. Thus, the dust and liquid compositions can be applied to the plant by the use of power-dusters, boom and hand sprayers and spray dusters. The compositions can also be applied from airplanes as a dust or a spray because they are effective in very low dosages. In order to modify or control growth of germinating seeds or emerging seedlings, as a typical example, the dust and liquid compositions are applied to the soil according to conventional methods and are distributed in the soil to a depth of at least 1/2 inch below the soil surface. It is not necessary that the phytotoxic compositions be admixed with the soil particles since these compositions can also be applied merely by spraying or sprinkling the surface of the soil. The phytotoxic compositions of this invention can also be applied by addition to irrigation water supplied to the field to be treated. This method of application permits the penetration of the compositions into the soil as the water is absorbed therein. Dust compositions, granular compositions or liquid formulations applied to the surface of the soil can be distributed below the surface of the soil by conventional means such as discing, dragging or mixing operations.

The phytotoxic compositions of this invention can also contain other additaments, for example, fertilizers, pesticides and the like, used as adjuvant or in combination with any of the abovedescribed adjuvants. Other phytotoxic compounds useful in combination with the abovedescribed compounds include, for example, 2,4-dichlorophenoxyacetic acids, 2,4,5-trichlorophenoxyacetic acid, 2-methyl-4-chlorophenoxyacetic acid and the salts, esters and amides thereof, triazine derivatives, such as 2,4-bis-(3-methoxypropylamino)-6-methylthio-s-triazine, 2-chloro-4-ethylamino-6-isopropylamino-s-triazine, and 2-ethylamino-4-isopropyl-amino-6-methyl-mercapto-s-triazine; urea derivatives, such as 3-(3,5-dichlorophenyl)-1,1-dimethylurea and 3-(p-chlorophenyl)-1,1-dimethylurea; and acetamides such as N,N-diallyl-α-chloroacetamide, and the like; benzoic acids such as 3-amino-2,5-dichlorogbenaoic acid; thiocarbamates such as S-propyl N,N-dipropytlthiocarbamate, S-ethyl N,N-dipropylthiocarbamate, S-ethyl cyclohexylethyl thiocarbamate, S-ethyl hexahydro-1H-azepine-1-carbothioate and the like; anilines such as 4-(methylsulfonyl)-2,6-dinitro-N,N-substituted aniline, 4-trifluoromethyl-2,6-dinitro-N,Ndi-n-propyl aniline and 4-trifluoromethyl-2,6-dinitro-N-ethyl-N,N-di-n-butyl aniline. Fertilizers useful in combination with the active ingredients include, for example, ammonium nitrate, urea and superphosphate. Other useful additaments include materials in which plant organisms take root and grow such as compost, manure, humus, sand, and the like.

**Claims**

**Claims for the following Contracting States : BE, CH, DE, FR, GB, IT, LI, NL, SE**

**1.** A compound having the structural formula

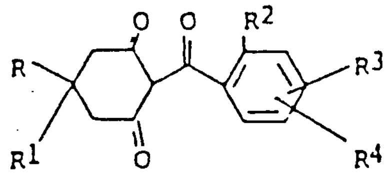

wherein

| | |
|---|---|
| R and $R^1$ | are hydrogen or $C_1$-$C_4$ alkyl; |
| $R^2$ | is chlorine, bromine or iodine; |
| $R^3$ | is hydrogen or halogen; and |
| $R^4$ | is hydrogen, chlorine, bromine or iodine, $C_1$-$C_4$ alkyl, $C_1$-$C_4$ alkoxy, nitro, or trifluoromethyl. |

**2.** The compound of Claim 1 wherein

| | |
|---|---|
| R and $R^1$ | are hydrogen or methyl; |
| $R^2$ | is chlorine, bromine, or iodine; |
| $R^3$ | is hydrogen; |
| $R^4$ | is hydrogen, chlorine, nitro or trifluoromethyl. |

**3.** The compound of Claim 2 wherein $R^4$ is substituted at the 4- or 5-position.

**4.** The compound of Claim 2 wherein

| | |
|---|---|
| R and $R^1$ | are hydrogen; |
| $R^2$ | is chlorine, bromine, or iodine; |
| $R^3$ | is hydrogen; and |
| $R^4$ | is 4-chlorine, 5-chlorine, 4-nitro, 5-$CF_3$ or hydrogen. |

**5.** The compound of Claim 4 wherein $R^2$ is chlorine.

**6.** The compound of Claim 1 wherein R is methyl, $R^1$ is methyl, $R^2$ is 2-chlorine, $R^3$ is hydrogen and $R^4$ is 6-chlorine.

**7.** The compound of Claim 1 wherein R is hydrogen, $R^1$ is hydrogen, $R^2$ is 2-chlorine, $R^3$ is hydrogen and $R^4$ is 4-chlorine.

**8.** The compound of Claim 1 wherein R is hydrogen, $R^1$ is hydrogen, $R^2$ is 2-chlorine, $R^3$ is hydrogen and $R^4$ is 6-chlorine.

**9.** The compound of Claim 1 wherein R is hydrogen, $R^1$ is hydrogen, $R^2$ is 2-chlorine, $R^3$ is 3-chlorine and $R^4$ is 6-chlorine.

**10.** The compound of Claim 1 wherein R is hydrogen, $R^1$ is hydrogen, $R^2$ is 2-chlorine, $R^3$ is hydrogen and $R^4$ is 5-trifluoromethyl.

**11.** The compound of Claim 1 wherein R is hydrogen, $R^1$ is hydrogen, $R^2$ is 2-chlorine, $R^3$ is 3-chlorine and $R^4$ is 4-chlorine.

**12.** The compound of Claim 1 wherein R is hydrogen, $R^1$ is hydrogen, $R^2$ is 2-chlorine, $R^3$ is 3-chlorine and $R^4$ is 4-methoxy.

**13.** The compound of Claim 1 wherein R is hydrogen, $R^1$ is hydrogen, $R^2$ is 2-chlorine, $R^3$ is hydrogen and $R^4$ is 4-bromine.

**14.** The method of controlling undesirable vegetation comprising applying to the area where control is

desired, an herbicidally effective amount of a compound having the formula

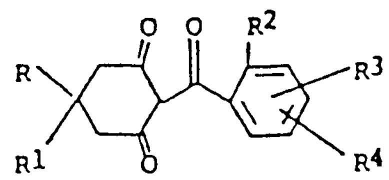

wherein

| | |
|---|---|
| R and $R^1$ | are hydrogen or $C_1$-$C_4$ alkyl; |
| $R^2$ | is chlorine, bromine, or iodine; |
| $R^3$ | is hydrogen or halogen; and |
| $R^4$ | is hydrogen, chlorine, bromine or iodine, $C_1$-$C_4$ alkyl, $C_1$-$C_4$ alkoxy, nitro, or trifluoromethyl. |

**15.** The method of Claim 14 wherein

| | |
|---|---|
| R and $R^1$ | are hydrogen or methyl; |
| $R^2$ | is chlorine, bromine, or iodine; |
| $R^3$ | is hydrogen; |
| $R^4$ | is hydrogen, chlorine, nitro or trifluoromethyl. |

**16.** The method of Claim 15 wherein $R^4$ is substitued at the 4- or 5-position.

**17.** The method of Claim 15 wherein

| | |
|---|---|
| R and $R^1$ | are hydrogen; |
| $R^2$ | is chlorine, bromine, or iodine; |
| $R^3$ | is hydrogen; and |
| $R^4$ | is 4-chlorine, 5-chlorine, 4-nitro, 5-$CF_3$ or hydrogen. |

**18.** The method of Claim 15 wherein $R^2$ is chlorine.

**19.** The method of Claim 14 wherein R is methyl, $R^1$ is methyl, $R^2$ is 2-chlorine, $R^3$ is hydrogen and $R^4$ is 6-chlorine.

**20.** The method of Claim 14 wherein R is hydrogen, $R^1$ is hydrogen, $R^2$ is 2-chlorine, $R^3$ is hydrogen and $R^4$ is 4-chlorine.

**21.** The method of Claim 14 wherein R is hydrogen, $R^1$ is hydrogen, $R^2$ is 2-chlorine, $R^3$ is hydrogen and $R^4$ is 6-chlorine.

**22.** The method of Claim 14 wherein R is hydrogen, $R^1$ is hydrogen, $R^2$ is 2-chlorine, $R^3$ is 3-chlorine and $R^4$ is 6-chlorine.

**23.** The method of Claim 14 wherein R is hydrogen, $R^1$ is hydrogen, $R^2$ is 2-chlorine, $R^3$ is hydrogen and $R^4$ is 5-trifluoromethyl.

**24.** The method of Claim 14 wherein R is hydrogen, $R^1$ is hydrogen, $R^2$ is 2-chlorine, $R^3$ is 3-chlorine and $R^4$ is 4-chlorine.

**25.** The method of Claim 14 wherein R is hydrogen, $R^1$ is hydrogen, $R^2$ is 2-chlorine, $R^3$ is 3-chlorine and $R^4$ is 4-methoxy.

**26.** The method of Claim 14 wherein R is hydrogen, $R^1$ is hydrogen, $R^2$ is 2-chlorine, $R^3$ is hydrogen and $R^4$ is 4-bromine.

**27.** A herbicidal composition comprising a compound having the structural formula

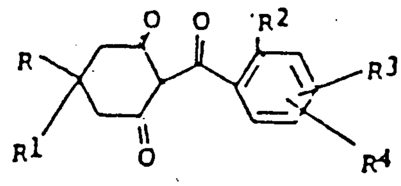

wherein

| | |
|---|---|
| R and $R^1$ | are hydrogen or $C_1$-$C_4$ alkyl; |
| $R^2$ | is chlorine, bromine, or iodine; |
| $R^3$ | is hydrogen or halogen; and |
| $R^4$ | is hydrogen, chlorine, bromine or iodine, $C_1$-$C_4$ alkyl, $C_1$-$C_4$ alkoxy, nitro or trifluoromethyl, |

and an inert carrier therefor.

**Claims for the following Contracting State : AT**

**1.** A process for preparing compounds having the structural formula I

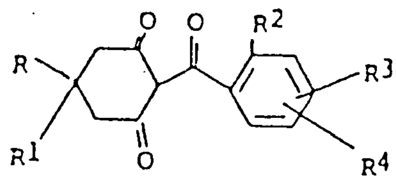

(I)

wherein

| | |
|---|---|
| R and $R^1$ | are hydrogen or $C_1$-$C_4$ alkyl; |
| $R^2$ | is chlorine, bromine, or iodine; |
| $R^3$ | is hydrogen or halogen; and |
| $R^4$ | is hydrogen, chlorine, bromine or iodine, $C_1$-$C_4$ alkyl, $C_1$-$C_4$ alkoxy, nitro, or trifluoromethyl, |

comprising reacting a substituted dione of the general formula II

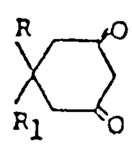

(II)

with a substituted benzoyl cyanide of the general formula III

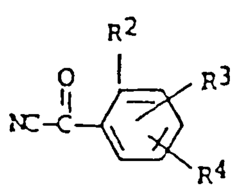

(III)

and working up the reaction product by conventional techniques.

**2.** The process of Claim 1 wherein

R and R $^1$ are hydrogen or methyl;
$R^2$ is chlorine, bromine, or iodine;
$R^3$ is hydrogen;
$R^4$ is hydrogen, chlorine, nitro or trifluoromethyl.

**3.** The process of Claim 2 wherein $R^4$ is substituted at the 4- or 5-position.

**4.** The compound of Claim 2 wherein
R and $R^1$ are hydrogen;
$R^2$ is chlorine, bromine, or iodine;
$R^3$ is hydrogen; and
$R^4$ is 4-chlorine, 5-chlorine, 4-nitro, 5-$CF_3$ or hydrogen.

**5.** The process of Claim 4 wherein $R^2$ is chlorine.

**6.** The process of Claim 1 wherein R is methyl, $R^1$ is methyl, $R^2$ is 2-chlorine, $R^3$ is hydrogen and $R^4$ is 6-chlorine.

**7.** The process of Claim 1 wherein R is hydrogen, $R^1$ is hydrogen, $R^2$ is 2-chlorine, $R^3$ is hydrogen and $R^4$ is 4-chlorine.

**8.** The process of Claim 1 wherein R is hydrogen, $R^1$ is hydrogen, $R^2$ is 2-chlorine, $R^3$ is hydrogen or 3-chlorine and $R^4$ is 6-chlorine, 5-trifluoromethyl, 4-chlorine, 4-methoxy, or 4-bromine.

**9.** The method of controlling undesirable vegetation comprising applying to the area where control is desired, an herbicidally effective amount of a compound having the formula

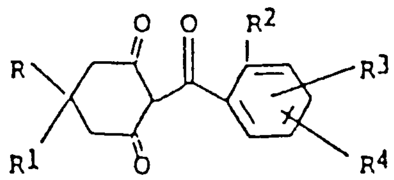

wherein
R and $R^1$ are hydrogen or $C_1$-$C_4$ alkyl;
$R^2$ is chlorine, bromine, or iodine;
$R^3$ is hydrogen, halogen; and
$R^4$ is hydrogen, chlorine, bromine or iodine, $C_1$-$C_4$ alkyl, $C_1$-$C_4$ alkoxy, nitro, or trifluoromethyl.

**10.** The method of Claim 9 wherein
R and $R^1$ are hydrogen or methyl;
$R^2$ is chlorine, bromine, or iodine;
$R^3$ is hydrogen;
$R^4$ is hydrogen, chlorine, nitro or trifluoromethyl and wherein $R^4$ is substituted at the 4- or 5-position.

**11.** The method of Claim 9 wherein
R and $R^1$ are hydrogen;
$R^2$ is chlorine, bromine, or iodine;
$R^3$ is hydrogen; and
$R^4$ is 4-chlorine, 5-chlorine, 4-nitro, 5-$CF_3$ or hydrogen.

**12.** The method of Claim 9 wherein
R is hydrogen or methyl;
$R^1$ is hydrogen or methyl;

$R^2$ is 2-chlorine;
$R^3$ is hydrogen or 3-chlorine; and
$R^4$ is 6-chlorine, 5-trifluoromethyl, 4-chlorine, 4-methoxy or 4-bromine.

**13.** A process for preparing a herbicidal composition comprising mixing a herbicidally active amount of a compound having the structural formula I

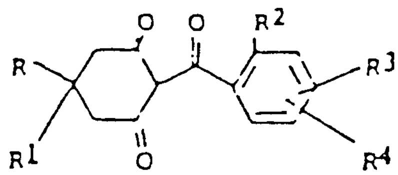

wherein
R and $R^1$ are hydrogen or $C_1$-$C_4$ alkyl;
$R^2$ is chlorine, bromine, or iodine;
$R^3$ is hydrogen, halogen; and
$R^4$ is hydrogen, chlorine, bromine or iodine, $C_1$-$C_4$ alkyl, $C_1$-$C_4$ alkoxy, nitro or trifluoromethyl,
and an inert carrier therefor.

**14.** A compound having the structural formula

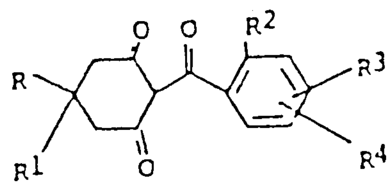

wherein
R and $R^1$ are hydrogen or $C_1$-$C_4$ alkyl;
$R^2$ is chlorine, bromine, or iodine;
$R^3$ is hydrogen, halogen; and
$R^4$ is hydrogen, chlorine, bromine or iodine, $C_1$-$C_4$ alkyl, $C_1$-$C_4$ alkoxy, nitro, or trifluoromethyl.

**15.** The compound of Claim 14 wherein
R and $R^1$ are hydrogen or methyl;
$R^2$ is chlorine, bromine, or iodine;
$R^3$ is hydrogen;
$R^4$ is hydrogen, chlorine, nitro or trifluoromethyl.

**16.** The compound of Claim 15 wherein $R^4$ is substituted at the 4- or 5-position.

**17.** The compound of Claim 15 wherein
R and $R^1$ are hydrogen;
$R^2$ is chlorine, bromine, or iodine;
$R^3$ is hydrogen; and
$R^4$ is 4-chlorine, 5-chlorine, 4-nitro, 5-$CF_3$ or hydrogen.

**18.** The compound of Claim 17 wherein $R^2$ is chlorine.

**19.** The compound of Claim 14 wherein R is methyl, $R^1$ is methyl, $R^2$ is 2-chlorine, $R^3$ is hydrogen and $R^4$ is 6-chlorine.

**20.** The compound of Claim 14 wherein R is hydrogen, $R^1$ is hydrogen, $R^2$ is 2-chlorine, $R^3$ is hydrogen and $R^4$ is 4-chlorine.

**21.** The compound of Claim 14 wherein R is hydrogen, $R^1$ is hydrogen, $R^2$ is 2-chlorine, $R^3$ is hydrogen and $R^4$ is 6-chlorine.

**22.** The compound of Claim 14 wherein R is hydrogen, $R^1$ is hydrogen, $R^2$ is 2-chlorine, $R^3$ is 3-chlorine and $R^4$ is 6-chlorine.

**23.** The compound of Claim 14 wherein R is hydrogen, $R^1$ is hydrogen, $R^2$ is 2-chlorine, $R^3$ is hydrogen and $R^4$ is 5-trifluoromethyl.

**24.** The compound of Claim 14 wherein R is hydrogen, $R^1$ is hydrogen, $R^2$ is 2-chlorine, $R^3$ is 3-chlorine and $R^4$ is 4-chlorine.

**25.** The compound of Claim 14 wherein R is hydrogen, $R^1$ is hydrogen, $R^2$ is 2-chlorine, $R^3$ is 3-chlorine and $R^4$ is 4-methoxy.

**26.** The compound of Claim 14 wherein R is hydrogen, $R^1$ is hydrogen, $R^2$ is 2-chlorine, $R^3$ is hydrogen and $R^4$ is 4-bromine.

**27.** A herbicidal composition comprising a compound having the structural formula

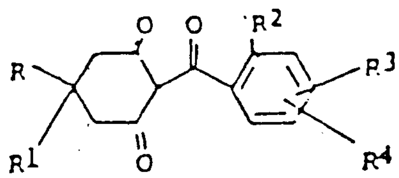

wherein

| | |
|---|---|
| R and $R^1$ | are hydrogen or $C_1$-$C_4$ alkyl; |
| $R^2$ | is chlorine, bromine, or iodine; |
| $R^3$ | is hydrogen, halogen; and |
| $R^4$ | is hydrogen, chlorine, bromine or iodine, $C_1$-$C_4$ alkyl, $C_1$-$C_4$ alkoxy, nitro or trifluoromethyl, |

and an inert carrier therefor.

**Revendications**

**Revendications pour les Etats contractants suivants : BE, CH, DE, FR, GB, IT, LI, NL, SE**

**1.** Un composé de formule structurelle: dans laquelle:

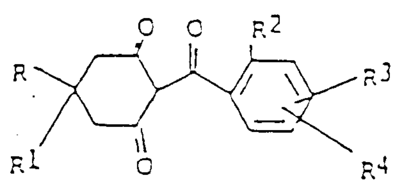

| | |
|---|---|
| R et $R^1$ | représentent un hydrogène ou un alkyle en $C_1$ à $C_4$; |
| $R^2$ | représente chlore, brome ou iode; |
| $R^3$ | représente l'hydrogène ou un halogène; et |
| $R^4$ | représente hydrogène, chlore, brome ou iode, alkyle en $C_1$ à $C_4$, alkoxy en $C_1$ à $C_4$, nitro ou trifluorométhyle. |

**2.** Le composé selon la revendication 1, dans lequel :

| | |
|---|---|
| R et $R^1$ | représentent un hydrogène ou un méthyle; |
| $R^2$ | représente chlore, brome ou iode; |
| $R^3$ | représente un hydrogène; |
| $R^4$ | représente hydrogène, chlore, nitro ou trifluorométhyle. |

**3.** Le composé selon la revendication 2, dans lequel $R^4$ est substitué en position 4 ou 5.

**4.** Le composé selon la revendication 2, dans lequel:

| | |
|---|---|
| R et $R^1$ | représentent l'hydrogène; |
| $R^2$ | représente chlore, brome ou iode; |
| $R^3$ | représente un hydrogène; et |
| $R^4$ | représente un chlore en position 4, un chlore en position 5, un nitro en position 4, un $CF_3$ en position 5 ou un hydrogène. |

**5.** Le composé selon la revendication 4, dans lequel $R^2$ représente le chlore.

**6.** Le composé selon la revendication 1, dans lequel R représente un méthyle, $R^1$ représente un méthyle, $R^2$ représente un chlore en position 2, $R^3$ représente l'hydrogène et $R^4$ représente un chlore en position 6.

**7.** Le composé selon la revendication 1, dans lequel R représente un hydrogène, $R^1$ représente un hydrogène, $R^2$ représente un chlore en position 2, $R^3$ représente un hydrogène et $R^4$ représente un chlore en position 6.

**8.** Le composé selon la revendication 1, dans lequel R représente un hydrogène, $R^1$ représente un hydrogène, $R^2$ représente un chlore en position 2, $R^3$ représente un hydrogène et $R^4$ représente un chlore en position 6.

**9.** Le composé selon la revendication 1, dans lequel R représente un hydrogène, $R^1$ représente un hydrogène, $R^2$ représente un chlore en position 2, $R^3$ représente un chlore en position 3 et $R^4$ représente un chlore en position 6.

**10.** Le composé selon la revendication 1, dans lequel R représente un hydrogène, $R^1$ représente un hydrogène, $R^2$ représente un chlore en position 2, $R^3$ représente un hydrogène et $R^4$ représente un trifluorométhyle en position 5.

**11.** Le composé selon la revendication 1, dans lequel R représente un hydrogène, $R^1$ représente un hydrogène, $R^2$ représente un chlore en position 2, $R^3$ représente un chlore en position 3 et $R^4$ représente un chlore en position 4.

**12.** Le composé selon la revendication 1, dans lequel R représente un hydrogène, $R^1$ représente un hydrogène, $R^2$ représente un chlore en position 2, $R^3$ représente un chlore en position 3 et $R^4$ représente un métoxy en position 4.

**13.** Le composé selon la revendication 1, dans lequel R représente un hydrogène, $R^1$ représente un hydrogène, $R^2$ représente un chlore en position 2, $R^3$ représente un hydrogène et $R^4$ représente un brome en position 4.

**14.** Le procédé de contrôle d'une végétation indésirable consistant à appliquer sur la zone où le contrôle est recherché une quantité efficace en tant qu'herbicide d'un composé ayant la formule:

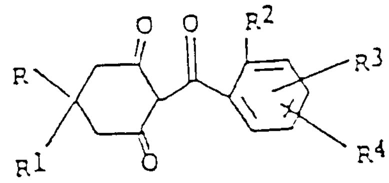

dans laquelle:

- R et $R^1$ représentent un hydrogène ou un alkyle en $C_1$ à $C_4$;
- $R^2$ représente chlore, brome ou iode;
- $R^3$ représente l'hydrogène ou un halogène; et
- $R^3$ représente hydrogène, chlore, brome ou iode, alkyle en $C_1$ à $C_4$, alkoxy en $C_1$ à $C_4$, nitro ou trifluorométhyle.

**15.** Le procédé selon la revendication 14, dans lequel R et $R^1$ représentent un hydrogène ou un méthyle, $R^2$ représente un chlore, brome ou iode, $R^3$ représente un hydrogène, $R^4$ représente hydrogène, chlore, nitro ou trifluorométhyle.

**16.** Le procédé selon la revendication 15, dans lequel $R^4$ est substitué en position 4 ou 5.

**17.** Le procédé selon la revendication 15, dans lequel:

- R et $R^1$ représentent un hydrogène;
- $R^2$ représente chlore, brome ou iode;
- $R^3$ représente un hydrogène; et
- $R^4$ représente un chlore en position 4, un chlore en position 5, un nitro en position 4, $CF_3$ en position 5 ou un hydrogène.

**18.** Le procédé selon la revendication 15, dans lequel $R^2$ représente un chlore.

**19.** Le procédé selon la revendication 14, dans lequel R représente un méthyle, $R^1$ représente un méthyle, $R^2$ représente un chlore en position 2, $R^3$ représente un hydrogène et $R^4$ représente un chlore en position 6.

**20.** Le procédé selon la revendication 14, dans lequel R représente un hydrogène, $R^1$ représente un hydrogène, $R^2$ représente un chlore en position 2, $R^3$ représente un hydrogène et $R^4$ représente un chlore en position 6.

**21.** Le procédé selon la revendication 14, dans lequel R représente un hydrogène, $R^1$ représente un hydrogène, $R^2$ représente un chlore en position 2, $R^3$ représente un hydrogène et $R^4$ représente un chlore en position 6.

**22.** Le procédé selon la revendication 14, dans lequel R représente un hydrogène, $R^1$ représente un hydrogène, $R^2$ représente un chlore en position 2, $R^3$ représente un chlore en position 3 et $R^4$ représente un chlore en position 6.

**23.** Le procédé selon la revendication 14, dans lequel R représente un hydrogène, $R^1$ représente un hydrogène, $R^2$ représente un chlore en position 2, $R^3$ représente un hydrogène et $R^4$ représente un trifluorométhyle en position 5.

**24.** Le procédé selon la revendication 14, dans lequel R représente un hydrogène, $R^1$ représente un hydrogène, $R^2$ représente un chlore en position 2, $R^3$ représente un chlore en position 3 et $R^4$ représente un chlore en position 4.

**25.** Le procédé selon la revendication 14, dans lequel R représente un hydrogène, $R^1$ représente un hydrogène, $R^2$ représente un chlore en position 2, $R^3$ représente un chlore en position 3 et $R^4$ représente un méthoxy en position 4.

**26.** Le procédé selon la revendication 14, dans lequel R représente un hydrogène, $R^1$ représente un hydrogène, $R^2$ représente un chlore en position 2, $R^3$ représente un hydrogène et $R^4$ représente un brome en position 4.

**27.** La composition herbicide constituée d'un composé de formule structurelle:

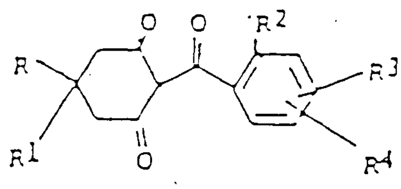

dans laquelle:

R et $R^1$ représentent un hydrogène ou un alkyle en $C_1$ à $C_4$;
$R^2$ représente chlore, brome ou iode;
$R^3$ représente l'hydrogène ou un halogène; et
$R^4$ représente hydrogène, chlore, brome ou iode, alkyle en $C_1$ à $C_4$, alkoxy en $C_1$ à $C_4$, nitro ou trifluorométhyle;

et d'un véhicule inerte vis-à-vis de ce composé.

**Revendications pour l'Etat contractant suivant : AT**

**1.** Le procédé de préparation de composés de formule structurelle I

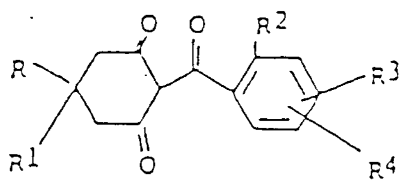

(I)

dans laquelle:

R et $R^1$ représentent un hydrogène ou un alkyle en $C_1$ à $C_4$;
$R^2$ représente chlore, brome ou iode;
$R^3$ représente l'hydrogène ou un halogène; et
$R^4$ représente hydrogène, chlore, brome ou iode, alkyle en $C_1$ à $C_4$, alkoxy en $C_1$ à $C_4$, nitro ou trifluorométhyle,

consistant à faire réagir une dione substituée de formule générale II

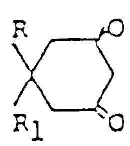

(II)

avec un cyanure de benzoyle substitué de formule générale III

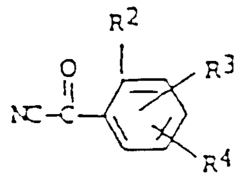

(III)

et à traiterle produit réactionnel par des techniques classiques.

**2.** Le procédé selon la revendication 1, dans lequel :

| | |
|---|---|
| R et $R^1$ | représentent un hydrogène ou un méthyle; |
| $R^2$ | représente chlore, brome ou iode; |
| $R^3$ | représente un hydrogène; |
| $R^4$ | représente hydrogène, chlore, nitro ou trifluorométhyle. |

**3.** Le procédé selon la revendication 2, dans lequel $R^4$ est substitué en position 4 ou 5.

**4.** Le composé selon la revendication 2, dans lequel :

| | |
|---|---|
| R et $R^1$ | représentent l'hydrogène; |
| $R^2$ | représente chlore, brome ou iode; |
| $R^3$ | représente un hydrogène; et |
| $R^4$ | représente un chlore en position 4, un chlore en position 5, un nitro en position 4, un $CF_3$ en position 5 ou un hydrogène. |

**5.** Le procédé selon la revendication 4, dans lequel $R^2$ représente le chlore.

**6.** Le procédé selon la revendication 1, dans lequel R représente un méthyle, $R^1$ représente un méthyle, $R^2$ représente un chlore en position 2, $R^3$ représente un hydrogène et $R^4$ représente un chlore en position 6.

**7.** Le procédé selon la revendication 1, dans lequel R représente un hydrogène, $R^1$ représente un hydrogène, $R^2$ représente un chlore en position 2, $R^3$ représente un hydrogène et $R^4$ représente un chlore en position 6.

**8.** Le procédé selon la revendication 1, dans lequel R représente un hydrogène, $R^1$ représente un hydrogène, $R^2$ représente un chlore en position 2, $R^3$ représente un hydrogène ou un chlore en position 3 et $R^4$ représente un chlore en position 6, un trifluorométhyle en position 5, un chlore en position 4, un méthoxy en position 4 ou un brome en position 4.

**9.** Le procédé de contrôle d'une végétation indésirable consistant à appliquer sur la zone où l'on recherche le contrôle une quantité efficace en tant qu'herbicide d'un composé de formule:

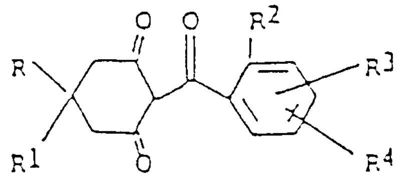

dans laquelle:

| | |
|---|---|
| R et $R^1$ | représentent un hydrogène ou un alkyle en $C_1$ à $C_4$; |
| $R^2$ | représente chlore, brome ou iode; |
| $R^3$ | représente l'hydrogène ou un halogène; et |
| $R^3$ | représente hydrogène, chlore, brome ou iode, alkyle en $C_1$ à $C_4$, alkoxy en $C_1$ à $C_4$, nitro ou trifluorométhyle. |

**10.** Le procédé selon la revendication 9, dans lequel :

R et $R^1$ représentent un hydrogène ou un méthyle;
$R^2$ représente chlore, brome ou iode;
$R^3$ représente un hydrogène;
$R^4$ représente un hydrogène, un chlore, un nitro ou un trifluorométhyle, et dans lequel $R^4$ est substitué en position 4 ou 5.

**11.** Le procédé selon la revendication 9, dans lequel:
R et $R^1$ représentent un hydrogène;
$R^2$ représente chlore, brome ou iode;
$R^3$ représente un hydrogène;
$R^4$ représente chlore en position 4-, chlore en position 5, nitro en position 4, $CF_3$ en position 5 ou un hydrogène.

**12.** Le procédé selon la revendication 9, dans lequel R représente un hydrogène ou un méthyle, $R^1$ représente un hydrogène ou un méthyle, $R^2$ représente un chlore en position 2, $R^3$ représente un hydrogène ou un chlore en position 3, $R^4$ représente un chlore en position 6, un trifluorométhyle en position 5, un chlore en position 4, un méthoxy en position 4 ou un brome en position 4.

**13.** Le procédé de préparation d'une composition herbicide consistant à mélanger une quantité active en tant qu'herbicide d'un composé de formule structurelle I

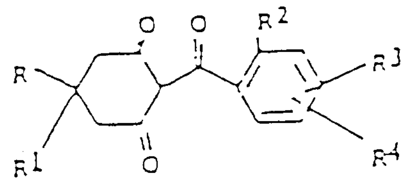

dans laquelle:
R et $R^1$ représentent un hydrogène ou un alkyle en $C_1$ à $C_4$;
$R^2$ représente chlore, brome ou iode;
$R^3$ représente l'hydrogène ou un halogène; et
$R^4$ représente hydrogène, chlore, brome ou iode, alkyle en $C_1$ à $C_4$, alkoxy en $C_1$ à $C_4$, nitro ou trifluorométhyle;
et un véhicule inerte vis-à-vis de ce compose.

**14.** Un composé de formule structurelle: dans laquelle:

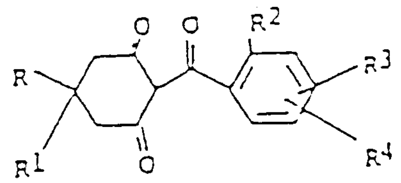

R et $R^1$ représentent un hydrogène ou un alkyle en $C_1$ à $C_4$;
$R^2$ représente chlore, brome ou iode;
$R^3$ représente l'hydrogène ou un halogène; et
$R^4$ représente hydrogène, chlore, brome ou iode, alkyle en $C_1$ à $C_4$, alkoxy en $C_1$ à $C_4$, nitro ou trifluorométhyle.

**15.** Le composé selon la revendication 1, dans lequel :
R et $R^1$ représentent un hydrogène ou un méthyle;
$R^2$ représente chlore, brome ou iode;
$R^3$ représente un hydrogène;

$R^4$ représente hydrogène, chlore, nitro ou trifluorométhyle.

**16.** Le composé selon la revendication 15, dans lequel $R^4$ est substitué en position 4 ou 5.

**17.** Le composé selon la revendication 15, dans lequel :
R et $R^1$ représentent l'hydrogène;
$R^2$ représente chlore, brome ou iode;
$R^3$ représente un hydrogène; et
$R^4$ représente un chlore en position 4, un chlore en position 5, un nitro en position 4, un $CF_3$ en position 5 ou un hydrogène.

**18.** Le composé selon la revendication 17, dans lequel $R^2$ représente le chlore.

**19.** Le composé selon la revendication 14, dans lequel R représente un méthyle, $R^1$ représente un méthyle, $R^2$ représente un chlore en position 2, $R^3$ représente l'hydrogène et $R^4$ représente un chlore en position 6.

**20.** Le composé selon la revendication 14, dans lequel R représente un hydrogène, $R^1$ représente un hydrogène, $R^2$ représente un chlore en position 2, $R^3$ représente un hydrogène et $R^4$ représente un chlore en position 6.

**21.** Le composé selon la revendication 14, dans lequel R représente un hydrogène, $R^1$ représente un hydrogène, $R^2$ représente un chlore en position 2, $R^3$ représente un hydrogène et $R^4$ représente un chlore en position 6.

**22.** Le composé selon la revendication 14, dans lequel R représente un hydrogène, $R^1$ représente un hydrogène, $R^2$ représente un chlore en position 2, $R^3$ représente un chlore en position 3 et $R^4$ représente un chlore en position 6.

**23.** Le composé selon la revendication 14, dans lequel R représente un hydrogène, $R^1$ représente un hydrogène, $R^2$ représente un chlore en position 2, $R^3$ représente un hydrogène et $R^4$ représente un trifluorométhyle en position 5.

**24.** Le composé selon la revendication 14, dans lequel R représente un hydrogène, $R^1$ représente un hydrogène, $R^2$ représente un chlore en position 2, $R^3$ représente un chlore en position 3 et $R^4$ représente un chlore en position 4.

**25.** Le composé selon la revendication 14, dans lequel R représente un hydrogène, $R^1$ représente un hydrogène, $R^2$ représente un chlore en position 2, $R^3$ représente un chlore en position 3 et $R^4$ représente un métoxy en position 4.

**26.** Le composé selon la revendication 14, dans lequel R représente un hydrogène, $R^1$ représente un hydrogène, $R^2$ représente un chlore en position 2, $R^3$ représente un hydrogène et $R^4$ représente un brome en position 4.

**27.** Une composition herbicide constituée d'un composé de formule structurelle:

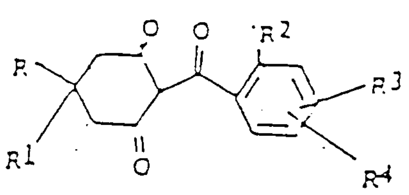

dans laquelle:
R et $R^1$ représentent un hydrogène ou un alkyle en $C_1$ à $C_4$;

$R^2$ représente chlore, brome ou iode;
$R^3$ représente l'hydrogène ou un halogène; et
$R^4$ représente hydrogène, chlore, brome ou iode, alkyle en $C_1$ à $C_4$, alkoxy en $C_1$ à $C_4$, nitro ou trifluorométhyle;

et d'un véhicule inerte vis-à-vis de ce composé.

**Patentansprüche**
**Patentansprüche für folgende Vertragsstaaten : BE, CH, DE, FR, GB, IT, LI, NL, SE**

**1.** Verbindung der Strukturformel:

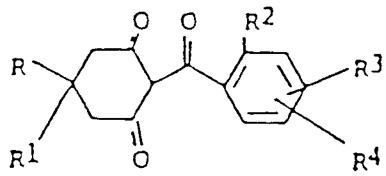

worin
R und $R^1$ Wasserstoff oder $C_1$-$C_4$-Alkyl bedeuten;
$R^2$ Chlor, Brom oder Iod bedeutet;
$R^3$ Wasserstoff oder Halogen bedeutet; und
$R^4$ Wasserstoff, Chlor, Brom oder Iod, $C_1$-$C_4$-Alkyl, $C_1$-$C_4$-Alkoxy, Nitro oder Trifluormethyl bedeutet.

**2.** Verbindung nach Anspruch 1, dadurch **gekennzeichnet,** daß
R und $R^1$ Wasserstoff oder Methyl;
$R^2$ Chlor, Brom oder Iod;
$R^3$ Wasserstoff;
$R^4$ Wasserstoff, Chlor, Nitro oder Trifluormethyl bedeuten.

**3.** Verbindung nach Anspruch 2, dadurch **gekennzeichnet,** daß $R^4$ in der 4- oder 5-Stellung substituiert ist.

**4.** Verbindung nach Anspruch 2, dadurch **gekennzeichnet,** daß
R und $R^1$ Wasserstoff;
$R^2$ Chlor, Brom oder Iod;
$R^3$ Wasserstoff; und
$R^4$ 4-Chlor, 5-Chlor, 4-Nitro, 5-$CF_3$ oder Wasserstoff bedeuten.

**5.** Verbindung nach Anspruch 4, dadurch **gekennzeichnet,** daß $R^2$ Chlor bedeutet.

**6.** Verbindung nach Anspruch 1, dadurch **gekennzeichnet,** daß R Methyl, $R^1$ Methyl, $R^2$ 2-Chlor, $R^3$ Wasserstoff und $R^4$ 6-Chlor bedeuten.

**7.** Verbindung nach Anspruch 1, dadurch **gekennzeichnet,** daß R Wasserstoff, $R^1$ Wasserstoff, $R^2$ 2-Chlor, $R^3$ Wasserstoff und $R^4$ 4-Chlor bedeuten.

**8.** Verbindung nach Anspruch 1, dadurch **gekennzeichnet,** daß R Wasserstoff, $R^1$ Wasserstoff, $R^2$ 2-Chlor, $R^3$ Wasserstoff und $R^4$ 6-Chlor bedeuten.

**9.** Verbindung nach Anspruch 1, dadurch **gekennzeichnet,** daß R Wasserstoff, $R^1$ Wasserstoff, $R^2$ 2-Chlor, $R^3$ 3-Chlor und $R^4$ 6-Chlor bedeuten.

**10.** Verbindung nach Anspruch 1, dadurch **gekennzeichnet,** daß R Wasserstoff, $R^1$ Wasserstoff, $R^2$ 2-Chlor, $R^3$ Wasserstoff und $R^4$ 5-Trifluormethyl bedeuten.

**11.** Verbindung nach Anspruch 1, dadurch **gekennzeichnet,** daß R Wasserstoff, $R^1$ Wasserstoff, $R^2$ 2-Chlor, $R^3$ 3-Chlor und $R^4$ 4-Chlor bedeuten.

**12.** Verbindung nach Anspruch 1, dadurch **gekennzeichnet,** daß R Wasserstoff, $R^1$ Wasserstoff, $R^2$ 2-Chlor, $R^3$ 3-Chlor und $R^4$ 4-Methoxy bedeuten.

**13.** Verbindung nach Anspruch 1, dadurch **gekennzeichnet,** daß R Wasserstoff, $R^1$ Wasserstoff, $R^2$ 2-Chlor, $R^3$ Wasserstoff und $R^4$ 4-Brom bedeuten.

**14.** Verfahren zur Kontrolle unerwünschter Vegetation, dadurch **gekennzeichnet,** daß auf die Fläche, wo eine Kontrolle erwünscht wird, eine herbizid wirksame Menge einer Verbindung der Formel:

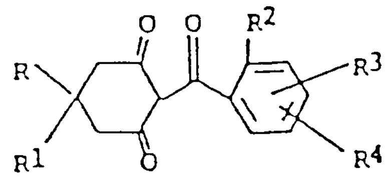

angewendet wird, worin

| | |
|---|---|
| R und $R^1$ | Wasserstoff oder $C_1$-$C_4$-Alkyl bedeuten; |
| $R^2$ | Chlor, Brom oder Iod bedeutet; |
| $R^3$ | Wasserstoff oder Halogen bedeutet; und |
| $R^4$ | Wasserstoff, Chlor, Brom oder Iod, $C_1$-$C_4$-Alkyl, $C_1$-$C_4$-Alkoxy, Nitro oder Trifluormethyl bedeutet. |

**15.** Verfahren nach Anspruch 14, dadurch **gekennzeichnet,** daß

| | |
|---|---|
| R und $R^1$ | Wasserstoff oder Methyl; |
| $R^2$ | Chlor, Brom oder Iod; |
| $R^3$ | Wasserstoff; |
| $R^4$ | Wasserstoff, Chlor, Nitro oder Trifluormethyl bedeuten. |

**16.** Verfahren nach Anspruch 15, dadurch **gekennzeichnet,** daß $R^4$ in der 4- oder 5-Stellung substituiert ist.

**17.** Verfahren nach Anspruch 15, dadurch **gekennzeichnet,** daß

| | |
|---|---|
| R und $R^1$ | Wasserstoff; |
| $R^2$ | Chlor, Brom oder Iod; |
| $R^3$ | Wasserstoff; und |
| $R^4$ | 4-Chlor, 5-Chlor, 4-Nitro, 5-$CF_3$ oder Wasserstoff bedeuten. |

**18.** Verfahren nach Anspruch 15, dadurch **gekennzeichnet,** daß $R^2$ Chlor bedeutet.

**19.** Verfahren nach Anspruch 14, dadurch **gekennzeichnet,** daß R Methyl, $R^1$ Methyl, $R^2$ 2-Chlor, $R^3$ Wasserstoff und $R^4$ 6-Chlor bedeuten.

**20.** Verfahren nach Anspruch 14, dadurch **gekennzeichnet,** daß R Wasserstoff, $R^1$ Wasserstoff, $R^2$ 2-Chlor, $R^3$ Wasserstoff und $R^4$ 4-Chlor bedeuten.

**21.** Verfahren nach Anspruch 14, dadurch **gekennzeichnet,** daß R Wasserstoff, $R^1$ Wasserstoff, $R^2$ 2-Chlor, $R^3$ Wasserstoff und $R^4$ 6-Chlor bedeuten.

**22.** Verfahren nach Anspruch 14, dadurch **gekennzeichnet,** daß R Wasserstoff, $R^1$ Wasserstoff, $R^2$ 2-Chlor, $R^3$ 3-Chlor und $R^4$ 6-Chlor bedeuten.

**23.** Verfahren nach Anspruch 14, dadurch **gekennzeichnet,** daß R Wasserstoff, $R^1$ Wasserstoff, $R^2$ 2-Chlor, $R^3$ Wasserstoff und $R^4$ 5-Trifluormethyl bedeuten.

**24.** Verfahren nach Anspruch 14, dadurch **gekennzeichnet,** daß R Wasserstoff, $R^1$ Wasserstoff, $R^2$ 2-Chlor, $R^3$ 3-Chlor und $R^4$ 4-Chlor bedeuten.

**25.** Verfahren nach Anspruch 14, dadurch **gekennzeichnet,** daß R Wasserstoff, $R^1$ Wasserstoff, $R^2$ 2-Chlor, $R^3$ 3-Chlor und $R^4$ 4-Methoxy bedeuten.

**26.** Verfahren nach Anspruch 14, dadurch **gekennzeichnet,** daß R Wasserstoff, $R^1$ Wasserstoff, $R^2$ 2-Chlor, $R^3$ Wasserstoff und $R^4$ 4-Brom bedeuten.

**27.** Herbizides Mittel, dadurch **gekennzeichnet,** daß es eine Verbindung der Strukturformel:

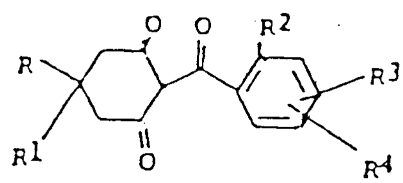

worin

| | |
|---|---|
| R und $R^1$ | Wasserstoff oder $C_1$-$C_4$-Alkyl bedeuten; |
| $R^2$ | Chlor, Brom oder Iod bedeutet; |
| $R^3$ | Wasserstoff oder Halogen bedeutet; und |
| $R^4$ | Wasserstoff, Chlor, Brom oder Iod, $C_1$-$C_4$-Alkyl, $C_1$-$C_4$-Alkoxy, Nitro oder Trifluormethyl bedeutet, und einen inerten Träger dafür enthält. |

**Patentansprüche für folgenden Vertragsstaat : AT**

**1.** Verfahren zur Herstellung von Verbindungen der Strukturformel I:

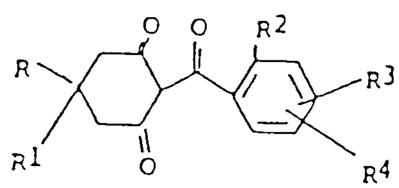

(I)

worin

| | |
|---|---|
| R und $R^1$ | Wasserstoff oder $C_1$-$C_4$-Alkyl bedeuten; |
| $R^2$ | Chlor, Brom oder Iod bedeutet; |
| $R^3$ | Wasserstoff oder Halogen bedeutet; und |
| $R^4$ | Wasserstoff, Chlor, Brom oder Iod, $C_1$-$C_4$-Alkyl, $C_1$-$C_4$-Alkoxy, Nitro oder Trifluormethyl bedeutet, |

dadurch **gekennzeichnet,** daß man ein substituiertes Dion der allgemeinen Formel II:

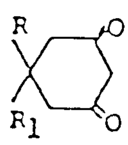

(II)

mit einem substituierten Benzoylcyanid der allgemeinen Formel III:

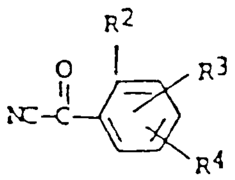

(III)

umsetzt und das Reaktionsprodukt nach an sich bekannten Verfahren aufarbeitet.

**2.** Verfahren nach Anspruch 1, dadurch **gekennzeichnet,** daß

| | |
|---|---|
| R und $R^1$ | Wasserstoff oder Methyl; |
| $R^2$ | Chlor, Brom oder Iod; |
| $R^3$ | Wasserstoff; |
| $R^4$ | Wasserstoff, Chlor, Nitro oder Trifluormethyl bedeuten. |

**3.** Verfahren nach Anspruch 2, dadurch **gekennzeichnet,** daß $R^4$ in der 4- oder 5-Stellung substituiert ist.

**4.** Verfahren nach Anspruch 2, dadurch **gekennzeichnet,** daß

| | |
|---|---|
| R und $R^1$ | Wasserstoff; |
| $R^2$ | Chlor, Brom oder Iod; |
| $R^3$ | Wasserstoff; und |
| $R^4$ | 4-Chlor, 5-Chlor, 4-Nitro, 5-$CF_3$ oder Wasserstoff bedeuten. |

**5.** Verfahren nach Anspruch 4, dadurch **gekennzeichnet,** daß $R^2$ Chlor bedeutet.

**6.** Verfahren nach Anspruch 1, dadurch **gekennzeichnet,** daß R Methyl, $R^1$ Methyl, $R^2$ 2-Chlor, $R^3$ Wasserstoff und $R^4$ 6-Chlor bedeuten.

**7.** Verfahren nach Anspruch 1, dadurch **gekennzeichnet,** daß R Wasserstoff, $R^1$ Wasserstoff, $R^2$ 2-Chlor, $R^3$ Wasserstoff und $R^4$ 4-Chlor bedeuten.

**8.** Verfahren nach Anspruch 1, dadurch **gekennzeichnet,** daß R Wasserstoff, $R^1$ Wasserstoff, $R^2$ 2-Chlor, $R^3$ Wasserstoff oder 3-Chlor und $R^4$ 6-Chlor, 5-Trifluormethyl, 4-Chlor, 4-Methoxy oder 4-Brom bedeuten.

**9.** Verfahren zur Kontrolle unerwünschter Vegetation, dadurch **gekennzeichnet,** daß auf die Fläche, wo eine Kontrolle erwünscht wird, eine herbizid wirksame Menge einer Verbindung der Formel:

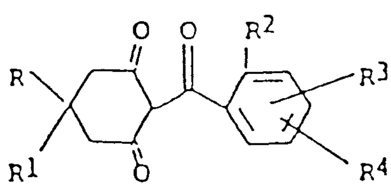

angewendet wird, worin

| | |
|---|---|
| R und $R^1$ | Wasserstoff oder $C_1$-$C_4$-Alkyl bedeuten; |
| $R^2$ | Chlor, Brom oder Iod bedeutet; |
| $R^3$ | Wasserstoff oder Halogen bedeutet; und |
| $R^4$ | Wasserstoff, Chlor, Brom oder Iod, $C_1$-$C_4$-Alkyl, $C_1$-$C_4$-Alkoxy, Nitro oder Trifluormethyl bedeutet. |

**10.** Verfahren nach Anspruch 9, dadurch **gekennzeichnet,** daß

| | |
|---|---|
| R und $R^1$ | Wasserstoff oder Methyl; |
| $R^2$ | Chlor, Brom oder Iod; |

R³ Wasserstoff;
R⁴ Wasserstoff, Chlor, Nitro oder Trifluormethyl, wobei $R^4$ in der 4- oder 5-Stellung substituiert ist, bedeuten.

**11.** Verfahren nach Anspruch 9, dadurch **gekennzeichnet,** daß
R und $R^1$ Wasserstoff;
$R^2$ Chlor, Brom oder Iod;
$R^3$ Wasserstoff; und
$R^4$ 4-Chlor, 5-Chlor, 4-Nitro, 5-$CF_3$ oder Wasserstoff
bedeuten.

**12.** Verfahren nach Anspruch 9, dadurch **gekennzeichnet,** daß
R Wasserstoff oder Methyl;
$R^1$ Wasserstoff oder Methyl;
$R^2$ 2-Chlor;
$R^3$ Wasserstoff oder 3-Chlor; und
$R^4$ 6-Chlor, 5-Trifluormethyl, 4-Chlor, 4-Methoxy oder 4-Brom
bedeuten.

**13.** Verfahren zur Herstellung eines herbiziden Mittels, dadurch **gekennzeichnet,** daß eine herbizid aktive Menge der Verbindung der Strukturformel I:

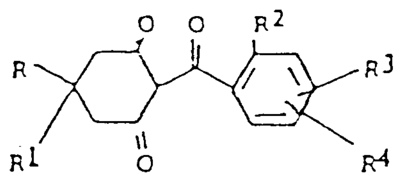

worin
R und $R^1$ Wasserstoff oder $C_1$-$C_4$-Alkyl bedeuten;
$R^2$ Chlor, Brom oder Iod bedeutet;
$R^3$ Wasserstoff oder Halogen bedeutet; und
$R^4$ Wasserstoff, Chlor, Brom oder Iod, $C_1$-$C_4$-Alkyl, $C_1$-$C_4$-Alkoxy, Nitro oder Trifluormethyl bedeutet,
und ein inerter Träger dafür vermischt werden.

**14.** Verbindung der Strukturformel:

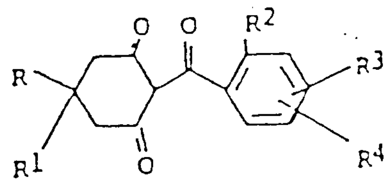

worin
R und $R^1$ Wasserstoff oder $C_1$-$C_4$-Alkyl bedeuten;
$R^2$ Chlor, Brom oder Iod bedeutet;
$R^3$ Wasserstoff oder Halogen bedeutet; und
$R^4$ Wasserstoff, Chlor, Brom oder Iod, $C_1$-$C_4$-Alkyl, $C_1$-$C_4$-Alkoxy, Nitro oder Trifluormethyl bedeutet.

**15.** Verbindung nach Anspruch 14, dadurch **gekennzeichnet,** daß

R und $R^1$ Wasserstoff oder Methyl bedeuten;
$R^2$ Chlor, Brom oder Iod bedeutet;
$R^3$ Wasserstoff bedeutet; und
$R^4$ Wasserstoff, Chlor, Nitro oder Trifluormethyl
bedeutet.

**16.** Verbindung nach Anspruch 15, dadurch **gekennzeichnet,** daß $R^4$ in der 4- oder 5-Stellung substituiert ist.

**17.** Verbindung nach Anspruch 15, dadurch **gekennzeichnet,** daß
R und $R^1$ Wasserstoff bedeuten;
$R^2$ Chlor, Brom oder Iod bedeutet;
$R^3$ Wasserstoff bedeutet; und
$R^4$ 4-Chlor, 5-Chlor, 4-Nitro, 5-$CF_3$ oder Wasserstoff bedeutet.

**18.** Verbindung nach Anspruch 17, dadurch **gekennzeichnet,** daß $R^2$ Chlor bedeutet.

**19.** Verbindung nach Anspruch 14, dadurch **gekennzeichnet,** daß R Methyl, $R^1$ Methyl, $R^2$ 2-Chlor, $R^3$ Wasserstoff und $R^4$ 6-Chlor bedeuten.

**20.** Verbindung nach Anspruch 14, dadurch **gekennzeichnet,** daß R Wasserstoff, $R^1$ Wasserstoff, $R^2$ 2-Chlor, $R^3$ Wasserstoff und $R^4$ 4-Chlor bedeuten.

**21.** Verbindung nach Anspruch 14, dadurch **gekennzeichnet,** daß R Wasserstoff, $R^1$ Wasserstoff, $R^2$ 2-Chlor, $R^3$ Wasserstoff und $R^4$ 6-Chlor bedeuten.

**22.** Verbindung nach Anspruch 14, dadurch **gekennzeichnet,** daß R Wasserstoff, $R^1$ Wasserstoff, $R^2$ 2-Chlor, $R^3$ 3-Chlor und $R^4$ 6-Chlor bedeuten.

**23.** Verbindung nach Anspruch 14, dadurch **gekennzeichnet,** daß R Wasserstoff, $R^1$ Wasserstoff, $R^2$ 2-Chlor, $R^3$ Wasserstoff und $R^4$ 5-Trifluormethyl bedeuten.

**24.** Verbindung nach Anspruch 14, dadurch **gekennzeichnet,** daß R Wasserstoff, $R^1$ Wasserstoff, $R^2$ 2-Chlor, $R^3$ 3-Chlor und $R^4$ 4-Chlor bedeuten.

**25.** Verbindung nach Anspruch 14, dadurch **gekennzeichnet,** daß R Wasserstoff, $R^1$ Wasserstoff, $R^2$ 2-Chlor, $R^3$ 3-Chlor und $R^4$ 4-Methoxy bedeuten.

**26.** Verbindung nach Anspruch 14, dadurch **gekennzeichnet,** daß R Wasserstoff, $R^1$ Wasserstoff, $R^2$ 2-Chlor, $R^3$ Wasserstoff und $R^4$ 4-Brom bedeuten.

**27.** Herbizides Mittel, dadurch **gekennzeichnet,** daß es eine Verbindung der Strukturformel:

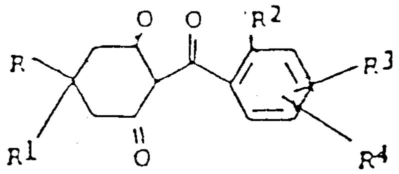

worin
R und $R^1$ Wasserstoff oder $C_1$-$C_4$-Alkyl bedeuten;
$R^2$ Chlor, Brom oder Iod bedeutet;
$R^3$ Wasserstoff oder Halogen bedeutet; und
$R^4$ Wasserstoff, Chlor, Brom oder Iod, $C_1$-$C_4$-Alkyl, $C_1$-$C_4$-Alkoxy, Nitro oder Trifluormethyl bedeutet,
und einen inerten Träger dafür enthält.